# EUROPEAN PATENT APPLICATION

(11) **EP 1 986 004 A2**
(43) Date of publication of application: **29.10.2008**
(21) Application number: 08155120.2
(22) Date of filing: 24.04.2008
(51) Int. Cl.: G01N 31/22, G01N 17/00

(54) **Chemical conversion film formation-evaluating liquid for steel**

(30) Priority: 26.04.2007 JP 2007117515
(71) Applicant: NHK Spring CO., LTD., Yokohama-shi, Kanagawa 236-0004 (JP)
(72) Inventor: Rikimaru, Taiichi, Kanagawa 236-0004 (JP); Suzuki, Takeshi, Kanagawa 236-0004 (JP); Ono, Yoshiki, Kanagawa 236-0004 (JP)
(74) Representative: Zech, Stefan Markus

(57) **Abstract**

A chemical conversion film formation-evaluating liquid for steel comprising an iron-dissolving liquid and a pH indicator, wherein pH value of the evaluating liquid is confined within the range of 4.0-8.0 with a lower limit of pH transition interval of the pH indicator being larger than the pH value, as a whole, of the evaluating liquid by a value of 0.1-3.0.

## Description

This invention relates to a chemical conversion film formation-evaluating liquid for steel for detecting chemically unconverted portions on the surface of steel that has been given a chemical conversion treatment.

Steel to be used in corrosive environments is frequently given a coating treatment for enhancing the corrosion resistance thereof. Further, in a situation where the corrosive environments are severe, the steel is given a chemical conversion treatment as a pretreatment of coating treatment for the purpose of enhancing the adhesion of coated film to the surface of steel or enhancing the corrosion resistance of steel after the coating treatment thereof. For example, zinc phosphate coating treatment has been widely employed in general up to date for past several tens years. However, there is a problem in this zinc phosphate coating treatment that because of various influences such as the stirring conditions, temperature and acidity ratio of treatment liquid and the adhesion of dusts onto the surface of steel, so-called chemical conversion film-lacking portion, i.e., a portion where the chemical conversion film is not formed thereon, is formed on the surface of steel.

This chemical conversion film-lacking portion is a portion where a coated film formed thereon can be easily peeled off, thus causing it to become a starting point of corrosion. As a result, the formation of this chemical conversion film-lacking portion would raise a great problem as it degrades the corrosion resistance of steel after the coating thereof. Therefore, the detection of the chemical conversion film-lacking portion of steel would lead to elucidation of the cause for the formation of defective film, thereby making it possible to take measures for the prevention of defective formation of film if, for example, the cause thereof resides in the process thereof.

However, since the normally formed portion of chemical conversion film and the defectively formed portion of chemical conversion film are alike in shade and figure, it has been difficult to visually distinguish them from each other even if the defectively formed portion of chemical conversion film is relatively large in size. Further, even if it is tried to observe the defectively formed portion of chemical conversion film by making use of an optical microscope or an electronic microscope, it is very difficult to perform the non-destructive inspection with regard to the existence of non-existence of such a defective portion throughout the entire surface of a complex three-dimensional body such, for example, as coil spring. Up to date, no one has succeeded in proposing a simple and practical evaluation method for detecting a defective portion of chemical conversion film.

By the way, a ferroxyl solution which is set forth in the claims of the present invention is known in the prior art as described in Appendix No. 3 of JIS H8617. This ferroxyl solution is designed to be employed for evaluating the pin-hole, etc., of plated articles. Further, a mixed solution comprising a ferroxyl solution and a phenolphthalein solution is known in the prior art as a ferroxyl indicator and described in a document "Anti-corrosion Technology", Vol. 30, pp. 580-587 (1981) (non-patent document). This non-patent document relates to the evaluation of the pretreatment (completeness of degreasing, etc.) which is based on the color development of ferroxyl indicator that may be caused to occur as the aforementioned mixed solution is applied drop-wise to the surface of steel and which is to be performed prior to the chemical conversion treatment of steel. However, this non-patent document fails to describe the mixing ratio between the ferroxyl solution and the phenolphthalein solution.

An object of the present invention is to provide a chemical conversion film formation-evaluating liquid for steel, which is capable of simply and easily detecting a defective portion of chemical conversion film where the chemical conversion film is not formed, the detection being based on the color development of the defective portion of chemical conversion film as the chemical conversion film is dipped in the evaluating liquid, thereby making it possible to evaluate the uniformity of surface treatment of steel.

This chemical conversion film formation-evaluating liquid for steel is characterized in that it comprises an iron-dissolving liquid, and a pH indicator, wherein pH value of the evaluating liquid is confined within the range of 4.0-8.0 with a lower limit of pH transition interval of the pH indicator being larger than the pH value, as a whole, of the evaluating liquid by a value of 0.1-3.0.

According to the present invention, it is now possible to simply and easily detect a defective portion of chemical conversion film where the chemical conversion film is not formed, the detection being based on the color development of the defective portion of chemical conversion film as the chemical conversion film is dipped in the evaluating liquid, thereby making it possible to evaluate the uniformity of treatment of the surface of steel.

The invention can be more fully understood from the following detailed description when taken in conjunction with the accompanying drawings, in which:
FIG. 1 is a perspective view for illustrating a situation wherein a defective portion of chemical conversion film is existed at a portion of the surface of steel according to Example 1 of the present invention;
FIG. 2 is a perspective view for illustrating a situation wherein a thin oxide film is formed on the surface of steel according to Example 2 of the present invention; and
FIG. 3 is a perspective view for illustrating a situation wherein a chemical conversion film consisting of zinc phosphate is formed all over the surface of steel according to Example 3 of the present invention.

Next, the present invention will be further explained in detail.

The method of evaluating a defective portion of chemical conversion film by making use of a chemical conversion film formation-evaluating liquid for steel according to the present invention is featured in that any defective portion of the chemical conversion film is detected on the basis of color development of the cathode portion that may be produced by an electrochemical reaction at a defective portion of the chemical conversion film in the drying stage to be performed subsequent to the dipping of the steel that has been already subjected to a chemical conversion treatment. More specifically, this method is featured in that, based on the following reaction formulas (1 and 2), the pH value of the evaluating liquid is caused to rise up to a pH value which exceeds the color development range of the pH indicator, thereby permitting the color to develop and that, based on this color development, a defective portion of chemical conversion film is detected. Reaction formula 1 represents a reaction through which iron component in the chemical conversion film is caused to elute as iron ion by making use of the evaluation liquid. Reaction formula 2 represents a reduction reaction that may be caused to occur at the defective portion of chemical conversion film by the effects of electrons released from iron.

Fe → Fe²⁺ + 2e⁻ (1)

2H₂O + O₂ + 4e⁻ → 4OH⁻ (2)

Next, there will be explained one example of electrochemical phenomenon which would take place in the drying stage subsequent to the dipping, in the evaluation liquid, of a steel member that has been subjected to a chemical conversion treatment and found accompanying a defective portion of chemical conversion film.

On the chemical conversion film acting as an anode as well as at the boundary of the defective portion, a very little amount of iron is caused to dissolve as the steel is contacted with the evaluating liquid, thereby enabling electrons to be released as shown by reaction formula 1. Especially, at a portion where residual strain is relatively large or at a portion where the elution of iron is relatively large such as the boundary of defective portion of chemical conversion film, iron ion is allowed to react with [Fe(CN)₆]⁴⁺ ion or [Fe(CN)₆]³⁺ ion included in the ferroxyl solution. As a result, a complex (Fe₂[Fe(CN)₆]Fe₃[Fe(CN)₆]₂Fe₄[Fe(CN)₆]₃) exhibiting a blue color is produced, thus developing blue color.

Meanwhile, at the defective portion of chemical conversion film acting as cathode, hydroxide ions are produced through a reducing reaction in which oxygen in water and air in the atmosphere as well as electrons released from iron are involved (reaction formula 2). Because of the production of hydroxide ions, the pH value of the evaluation liquid existing in the vicinity of the defective portion is caused to rise, exceeding the pH transition interval of pH8-10, thus developing pink color.

The evaluation method to be provided by the evaluation liquid according to the present invention is characterized in that the aforementioned color development of pink color is detected as a defective portion of chemical conversion film. In the case where a pH indicator other than phenolphthalein is employed, the defective portion of chemical conversion film can be evaluated based on a discoloration which is peculiar to such a pH indicator. As for the color development of blue color, it is a phenomenon which can be derived as a ferroxyl solution set forth in claims 2 and 3 is employed. In the case where the evaluation liquid to be provided according to claim 1 is employed, the phenomenon to be derived therefrom may not necessarily be the development of color and also may not necessarily be required for the evaluation of the defective portion of chemical conversion film.

On the other hand, when the steel which is free form any defective portion of chemical conversion film is dipped in the evaluation liquid of the present invention and then dried, a local cell cannot be created because of the fact that the surface of the steel is completely covered with a chemical conversion film. For this reason, the amount of elution of iron taking part in the anode reaction is very small and hence the electrons to be released are very small in quantity. Further, since the cathode portion where the electrons that have been released concentrates does not exist, the density of generating the reducing reaction is weakened. For this reason, there are no possibilities of developing pink color which can be visually recognized. Therefore, if there is no color development of pink color, it can be determined that any defective portion of chemical conversion film which is substantially detrimental is not existed on the surface of the steel.

In the aforementioned chemical conversion film formation-evaluating liquid for iron, the iron-dissolving liquid may be a ferroxyl solution, and the pH indicator may be formed of at least one kind of compound selected from phenol red, neutral red, p-α-naphtholphthalein, cresol red, thymol blue, and phenolphthalein, wherein the concentration of the pH indicator in the evaluating liquid is confined within the range of 0.1-1g per liter of aqueous solution.

In the present invention, the pH value of the evaluating liquid is regulated to the range of 4.0 to 8.0 because of the following reasons. Namely, when an evaluating liquid whose pH value is lower than 4.0 is employed, a normally formed chemical conversion film may be also damaged, thus making it impossible to accurately evaluate the chemical conversion film. On the other hand, when the pH value of the evaluating liquid becomes higher, the dissolution of iron component would become difficult. Therefore, the pH value of the evaluating liquid should preferably be limited to not higher than 8.0 in practical viewpoints.

In the present invention, the lower limit of the transition interval of pH indicator is regulated higher than the pH value of the evaluation liquid by 0.1 to 3.0 because of the following reasons. Namely, when the lower limit of the transition interval of pH indicator is lower than 0.1, the discoloration may be caused to occur whenever the pH value is raised to a small extent even before the application of the evaluating liquid. On the other hand, when the lower limit of the transition interval of pH indicator is higher than 3.0, the development of pink color for detecting the defects (cathode) can hardly take place, thus making it undesirable in practical viewpoint.

In the present invention, the concentration of the pH indicator in the evaluating liquid is regulated to the range of 0.1 to 1g per liter of aqueous solution because of the following reasons. Namely, when the concentration of the pH indicator is lower than 0.1g/L(gram/litter), it may become difficult to detect the defects by way of color development. On the other hand, when the concentration of the pH indicator is higher than 1g/L, the pH indicator cannot be completely dissolved in the ferroxyl solution, resulting in an increase in quantity of precipitation of the pH indicator, hence resulting in economical waste, and permitting the insoluble matter to adhere to the surface of chemical conversion film (a subject material to be evaluated), thereby making it difficult to evaluate the chemical conversion film.

### Examples

Followings are specific examples, which are not intended to restrict the scope of the present invention.

The evaluation liquid employed herein was an aqueous solution exhibiting a pH value of 6.1 and consisting of a mixed solution comprising a ferroxyl solution and a phenolphthalein solution, which are mixed at a volume ratio of 10:1. This ferroxyl solution was formulated so as to be constituted by pure water as a solvent, 10g/L of potassium pherrocyanide, 10g/L of potassium pherricyanide, and 60g/L of sodium chloride. This phenolphthalein solution was formulated so as to be constituted by 95% ethanol as a solvent, and 1g/L of phenolphthalein powder.

The subject material to be evaluated was a steel coil spring (type of steel: SAE 9254; wire: 12 mm diameter; outer diameter of coil: 122.5 mm; free height: 382 mm). Main manufacturing steps were: hot coiling; shot peening; and zinc phosphate coating treatment. This coil spring was dipped in the aforementioned evaluation liquid for 5 seconds and then allowed to dry in an air atmosphere for 10 minutes, after which the resultant chemical conversion film was evaluated with regard to any defects.

### (Example 1)

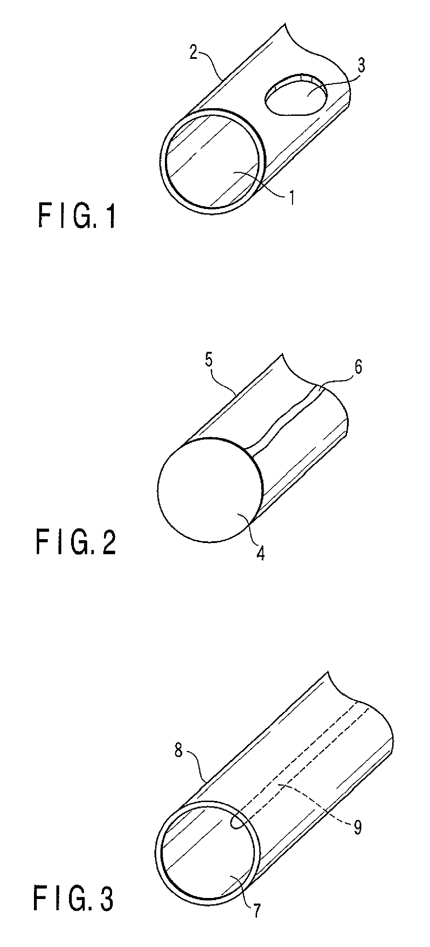

FIG. 1 shows a portion of steel coil spring for illustrating a situation wherein a defective portion of chemical conversion film was intentionally formed thereon by masking it after the step of shot peening. As shown in FIG. 1, a chemical conversion film 2 consisting of zinc phosphate was formed substantially all over the surface of steel 1 by means of zinc phosphate coating treatment. Further, a thin oxide film 3 which was formed by means of masking subsequent to the step of shot peening was left on a surface portion of the steel 1.

The cross-sectional surface of wire shown herein schematically illustrates the construction of wire consisting of the chemical conversion film and the steel, so that, as a matter of fact, the end face of the wire was also covered with the chemical conversion film. When this steel coil spring was dipped in the evaluation liquid and then dried, the development of color was not recognized on most of the surface of wire. However, a portion of the surface of wire went blue and the portion which was not subjected to the chemical conversion treatment because of the existence of the masking went pink. The reason for the development of the blue color could be explained as follows. Namely, the surface of chemical conversion film acting optionally as an anode as well as the boundary portion between the normal portion of chemical conversion film and the defective portion thereof was liable to create a local cell, thereby enabling the eluted iron ion to concentrate in quantity at these portions. As a result, these portions were assumably permitted to develop blue color through a reaction between the iron ion and the ferroxyl solution which was a component of the pH indicator.

On the other hand, the portion where the oxide film was left remain (a defective portion of chemical conversion film) because of the existence of the masking was enabled to act as a cathode relative to the other portion. Then, because of the reducing reaction where oxygen in water and air in the atmosphere as well as electrons released from iron are involved, hydroxide ions were produced. As a result, presumably because of the reaction between the hydroxide ions and the phenolphthalein solution which was a component of the evaluation liquid, a pink color developed. As can be clearly seen from the above results, since only the defective portion where the chemical conversion film does not exist can be selectively turned pink, a defective portion of the chemical conversion film can be easily detected using the development of a pink color as a criterion.

According to above Example 1, since only a defective portion of a chemical conversion film is enabled to develop a pink color by simply dipping a steel member in the aforementioned evaluating liquid, the defective portion can be detected in a simple and easy manner.

### (Example 2)

FIG. 2 shows a portion of steel coil spring for illustrating a situation wherein the surface of steel 4 is not provided with a chemical conversion film and, as seen from the entire surface of steel 4, the steel 4 that has been subjected to shot peening is left as it is. In this case, the surface of steel 4 would be immediately turned into a thin oxide film 5.

The cross-sectional surface of wire shown herein simply illustrates the construction of wire consisting of the oxide film and the steel, so that, as a matter of fact, the end face of the wire was also covered with the oxide film. In this case, as shown in FIG. 2 for example, a tool trace 6 is caused to be partially created on the occasion of coiling process. In this case, the surface portion accompanied with this tool trace 6 is relatively large in residual strain, so that iron is enabled to be eluted more easily therefrom as compared with other surface portions.

When this coil spring having the aforementioned features was dipped in the evaluation liquid and then dried, the development of a blue color was recognized mainly in the region where the tool trace 6 existed and most of the area other than the region where the tool trace 6 existed was turned pink. The reason for this phenomenon may be assumably attributed to the fact that even if there was uniformly formed a thin oxide film on the surface of wire, most of the surface of wire other than the region of tool trace which was relatively large in residual strain was turned into a cathode, thereby enabling most of the surface to turn into pink color. As seen from this result, even if most of the surface area is constituted by a defective chemical conversion film, a defective portion of chemical conversion film can be easily detected with the development of pink color being utilized as a criterion.

According to above Example 2, even if zinc phosphate chemical conversion film was not formed all over the surface area, most of the surface area other than the region where the tool trace 6 was existed was enabled to turn into pink color. Therefore, a defective portion of chemical conversion film could be easily detected as in the case of Example 1.

### (Example 3)

FIG. 3 shows a case wherein a chemical conversion film 8 consisting of zinc phosphate was formed all over the surface of steel 7 by means of zinc phosphate coating treatment. A portion of steel 7 was accompanied with a tool trace 9 which was formed on the occasion of coiling treatment of a coil spring and hence this portion of steel 7 was relatively large in residual strain. The cross-sectional surface of wire shown herein schematically illustrates the construction of wire consisting of the chemical conversion film and the steel, so that, as a matter of fact, the end face of the wire was also covered with the chemical conversion film.

When this steel coil spring was dipped in the evaluation liquid and then dried, the development of blue color was recognized only partially in the form of dots. The reason for this could be assumably explained as follows. Namely, the chemical conversion film was accompanied, even though very small in magnitude, with non-uniformity in iron component or in residual stain. Thus, the development of pink color was not recognized. The reason for this may be attributed to the following facts. Namely, since the zinc phosphate chemical conversion film 8 was formed all over the surface of coil spring, the quantity eluted of iron was very small throughout the entire surface including a region where tool trace 9 was existed and hence the quantity released of electrons was also very small. Furthermore, since there was not existed a defective portion acting as a cathode where these electrons were enabled to get together concentratedly, there was almost no room for the reducing reaction to take place, thus failing to recognize the development of pink color.

Further, properly speaking, if the development of blue color is recognized at even a small portion of the surface, it is conceivable that the development of pink color (cathode) may be existed somewhere in view of the principle of local cell reaction. However, since the development of blue color was very small, the development of pink color was assumably too small to discriminate it from others. In view of above results, it was determined that since there was no development of pink color, any kind of defective chemical conversion film that might degrade the performance of coil spring in practical viewpoint was not existed on the surface of coil spring.

According to above Example 3, it was determined that if there was no development of pink color in a situation wherein zinc phosphate chemical conversion film was formed all over the surface of steel without accompanying any defective portion, there was not existed any kind of defective chemical conversion film that might degrade the performance of coil spring in practical viewpoint.

By the way, it should not be construed that the present invention is limited by these examples but should be understood that the present invention can be variously modified in the practical application thereof without departing from the scope of the general inventive concept as defined by the appended claims. Further, a plurality of constituent elements disclosed in these examples may be optionally combined to create various forms of invention. For example, some of constituent elements may be omitted from the entire constituent elements disclosed in these embodiments. Furthermore, the constituent elements described in different examples may be optionally combined.

## Claims

1. A chemical conversion film formation-evaluating liquid for steel **characterized by** comprising an iron-dissolving liquid and a pH indicator, wherein pH value of the evaluating liquid is confined within the range of 4.0-8.0 with a lower limit of pH transition interval of the pH indicator being larger than the pH value, as a whole, of the evaluating liquid by a value of 0.1-3.0.

2. The chemical conversion film formation-evaluating liquid for steel according to claim 1, **characterized in that** the iron-dissolving liquid is a ferroxyl solution; and the pH indicator is formed of at least one kind of compound selected from phenol red, neutral red, p-α-naphtholphthalein, cresol red, thymol blue, and phenolphthalein; wherein the concentration of the pH indicator in the evaluating liquid is confined within the range of 0.1-1g per liter of aqueous solution.

3. The chemical conversion film formation-evaluating liquid for steel according to claim 2, **characterized in that** the pH indicator is formed of phenolphthalein.
